# EUROPEAN PATENT APPLICATION

(11) **EP 0 832 656 A1**
(43) Date of publication of application: **01.04.1998**
(21) Application number: 96115649.4
(22) Date of filing: 30.09.1996
(51) Int. Cl.: A61M 1/34, A61M 1/30

(54) **Method and apparatus for controlling the fluid balance of patients subjected to single-needle blood treatments**

(71) Applicant: B. BRAUN CAREX S.p.A., 41037 Mirandola (Modena) (IT)
(72) Inventor: Ferri, Angelo, 41037 Mirandola (IT); Borra, Mirco, 41019 Soliera (IT)
(74) Representative: Modiano, Guido, Dr.-Ing.

(57) **Abstract**

During atlernating collection and reinfusion phases, the filtration pressure varies cyclically between a minimum value and a maximum value. These value controls the length of these phases. Fluid balance is controlled by changing the average of these values, without using a specific pump for this purpose.

## Description

The present invention relates to a method and an apparatus for controlling the fluid balance of patients subjected to single-needle hemodialysis, hemofiltration and hemodiafiltration treatments.

In performing hemodialysis, hemofiltration, and hemodiafiltration therapies it is indispensable to be able to program each patient's acceptable weight loss caused by the evacuation of fluids in order to restore the correct body fluid balance and simultaneously not to expose the patient to the risk of volemic disorders (hypo-hypervolemia).

This loss is normally programmed by the attending practitioner according to a method that is known in the field of hemodialysis and is already used by some researchers: the practitioner in practice uses special machines having a programmed operating electronic logic system that can be modified for each patient; the practitioner enters a plurality of personal data and presets the weight loss to be achieved for each treatment as well as the total time over which said loss must be completed.

This is currently achieved by using apparatuses which are substantially constituted by a plurality of lines with tubes that feed the blood to be purified, which is taken from the patient, to a filter and return the blood to the patient at the outlet of said filter.

Corresponding peristaltic pumps are placed along these lines and move the blood of the patient along said lines; in particular, at least one specifically provided pump is assigned to so-called ultrafiltration: in practice, it is connected to the filter, and by being activated on command from a device that automatically checks the amount of filtrate extracted from the patient's body, it increases the negative pressure, and therefore the transmembrane pressure, inside the filter, so that said filter accordingly increases its filtration capacity when the filtrate is insufficient with respect to the data set by the medical operator.

The need to arrange specifically provided pump along these lines to perform said ultrafiltration function entails a significant increase in the costs of these apparatuses, in addition to a greater constructive complexity thereof.

A principal aim of the present invention is to solve the above described problems of the prior art, by providing a method and an apparatus for controlling the fluid balance of patients subjected to single-needle hemodialysis, hemofiltration and hemodiafiltration treatments that allows to control so-called ultrafiltration automatically but without using a specifically provided pump.

This aim and other objects are achieved by a method for controlling the fluid balance of a patient subjected to single-needle hemodialysis, which comprises a first step for extracting blood to be purified from the patient, followed by a second step for the filtration and weight-based collection of the filtrate and by a third step for returning the purified blood to the patient, characterized in that during said second filtration step the average filtration pressure (transmembrane pressure) can be changed on command from a normal value to an ultrafiltration value and vice versa, said average value being in turn correlatable with a treatment completion time, a programmed weight change of the patient, said average pressure value being the result of corresponding limit values, a higher value and a lower value, which can be preset as required in an associated electronic logic control unit.

Advantageously, the apparatus for performing the method comprises a first line for extracting blood from a patient through a single vascular access point; said first line is controlled by a first peristaltic pump that operates intermittently and sends said blood cyclically to a filter, from which it flows out purified so as to be returned to the patient through a second infusion line, arranged in series to said first line and leading into said vascular access, and which is controlled by a second peristaltic pump that operates intermittently in strict alternation with respect to said first pump; at least one third line is also connected to said filter in order to discharge the filtrate into containers that can be coupled to a unit for weighing infusion or dialysis liquid containers and are also coupled to said unit and are connected to said first extraction line; characterized in that a means for activating the alternation in the operation of said first and second pumps is interposed between said filter and said second pump, said means being adapted to control a pressure value, which is the average between two presettable values, respectively a minimum value and a maximum value, inside said filter, modulating its flow-rate from the normal value to the ultrafiltration value and vice versa.

Further characteristics and advantages of the invention will become apparent from the following detailed description of a preferred embodiment of a method and an apparatus for controlling the fluid balance of a patient subjected to single-needle hemodialysis treatment, said apparatus being illustrated only by way of non-limitative example in the accompanying drawings, wherein:

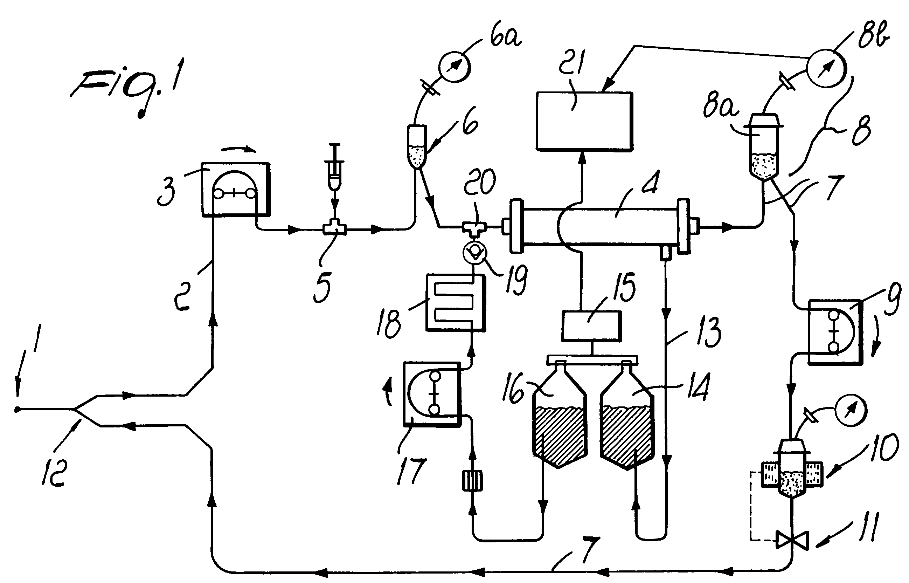

figure 1 is a schematic view of the functional circuits of an apparatus for performing a method for controlling the fluid balance of a patient subjected to single-needle hemofiltration, according to the present invention;

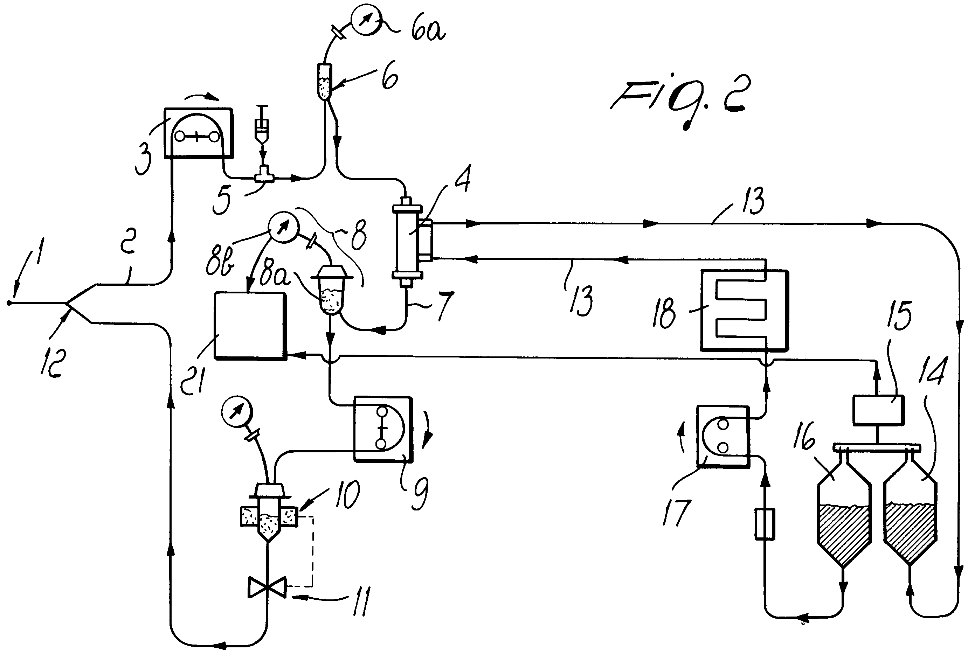

figure 2 is another schematic view of the same apparatus for performing the method according to the present invention, for a patient subjected to hemodiafiltration;

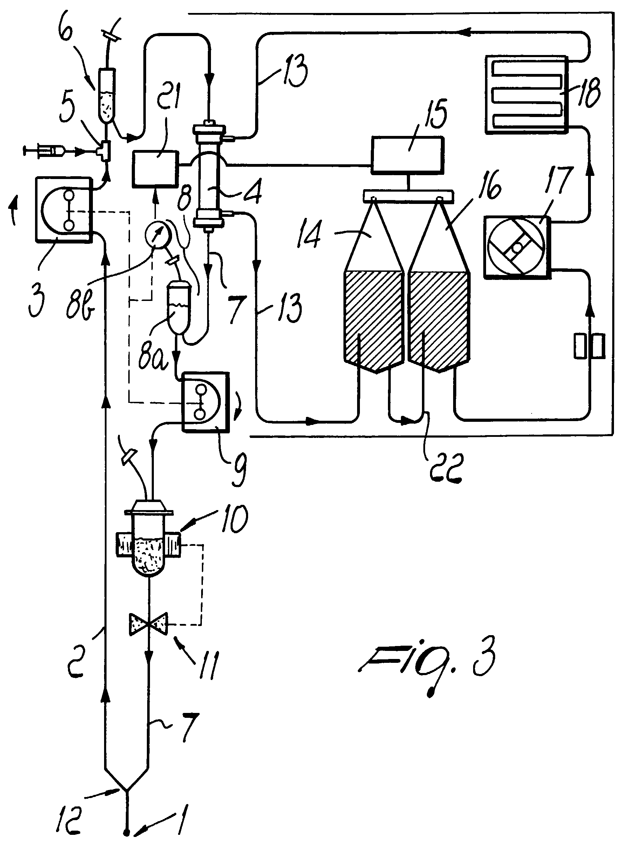

figure 3 is a further schematic view of the same apparatus for performing the method according to the present invention, for a patient subjected to hemodialysis.

With particular reference to the above figures, the reference numeral 1 generally designates a vascular access point of a patient.

A first blood extraction line 2 leads out from said vascular access point 1 and the blood is conveyed towards a filter 4 by a first peristaltic pump 3.

Pierceable access means 5 and means 6 for connection to instruments 6a adapted to measure the pressure inside said first line 2 are usually provided between the pump 3 and the filter 4 but are not indispensable.

A second line 7 for reinfusing the purified blood to the patient leads out of the filter 4; a means 8 for activating the alternation in the operation of the first pump 3 and of a second pump 9 is arranged along said second line, said second pump being adapted to provide a pressure that is sufficient for said reinfusion and is arranged downstream of said means 8, which is described more clearly hereinafter.

At least one device 10 for detecting air in the second line 7 and clamp means 11 with an alternating cyclic operation, closely in step with the active step of the second pump 9, are also provided downstream of the second pump 9.

The end part of the second line 7 leads into a conventional Y-shaped union, designated by the reference numeral 12, through which it reaches the vascular access point 1.

A third line 13 also leads out of the filter 4 and carries the residue, known as filtrate, to a container 14 that is suspended from a weighing unit 15 and is counterbalanced by a second container 16, in which a replacement or dialysis solution, as usually defined by the expert in the field, is placed.

From said second container, the third line 13 passes through a third peristaltic pump 17, a heating element 18, and optionally a one-way valve 19, and then joins the first line 2 again, either directly upstream of the filter 4, in a so-called predilution configuration, by means of a conventional union 20, or downstream of said filter, in a so-called postdilution configuration.

Said means 8 substantially comprises a hermetic chamber 8a, inside which a volume of incompressible liquid and a volume of air are placed in a dynamic equilibrium; said hermetic chamber 8a is furthermore connected to a pressure detector 8b, which sends its findings to a control processor generally designated by the reference numeral 21.

The operation of the invention is as follows:
the blood is extracted from the patient through the single vascular access point 1 and drawn by means of the first pump 3 along the first line 2.

During the action of the first pump 3, the second pump 9 is inactive and the clamp element 11 is closed.

By flowing along the line 2, the blood reaches the filter 4; in the portion lying upstream of said filter 4 it is possible to install access means 5, which in practice consist of a union provided with a pathway for connection, through a connecting tube, to a container for a supplementary drug, and means 6 for connection to an instrument 6a adapted to detect the pressure in said line 2.

Downstream of the filter 4, the blood enters the second line 7 and is conveyed, now purified, to the patient, passing through the hermetic chamber 8a, inside which a volume of said blood is kept in dynamic equilibrium with a complementary volume of air; the pressure inside the chamber 8a varies cyclically between a maximum value and a minimum value which are preset by the operator at the beginning of the cycle; the operator chooses a given average value and the instrument 8b (pressure transducer) activates, on the basis of the achieved limit values, the alternating startup of the pump 3 or of the pump 9, which returns the purified blood to the patient.

The filter 4 is also connected to the weighing unit 15 by means of the third line 13; this unit maintains a constant equilibrium between the filtrate container 14 and the container 16 of the infusion solution, which reaches the union 20, by means of the third pump 17, which operates continuously, by passing through the heater 18, entering the line 2 directly upstream of the filter 4, in a so-called predilution configuration (downstream in the case of postdilution); this equilibrium is determined by the weight loss that is set by the medical operator when the treatment begins.

Downstream of the second pump 9, before the blood reaches the patient, along the second line 7, there is provided a device 7 for detecting air in said line and a clamp means 11 is also provided, which is cyclically active in phase opposition with respect to the active phase of the second pump 9; the clamp means prevents the reverse flow of the blood towards the patient when the second pump 9 is deactivated.

The above-described cycle occurs in normal operating conditions; if an imbalance occurs with respect to the weight loss that was programmed for the patient, and therefore an imbalance between the containers 14 and 16, so that the filtrate is less than the expected amount, the weighing unit 15 sends this information to the processor 21, which promptly corrects the average pressure value to be produced inside the hermetic chamber 8a and accordingly modifies, by a constant and common value, the limit pressure values at which the pressure transducer 8b activates the cyclic mutual reversal of the intervention of the pumps 3 and 9.

The delay in the disengagement of the pump 3 produces an overpressure inside the filter 4, which accordingly increases its filtration capacity: this leads to a corresponding increase in the filtrate, known as "ultrafiltrate", which deposits in its own container 14, thus rebalancing the operation of the apparatus and returning it to the programmed steady state; the entire process occurs without requiring a pump that is provided specifically to achieve ultrafiltration control.

If the apparatus is used for hemodialysis, the containers 14 and 16 can be interconnected by a duct 22 and both contain a sterile dialysis solution; in this case, the third line 13 reenters the filter 4 and the filtrate is gradually removed from said filter with a repetitive plurality of passes of said solution, causing the overall weight of the containers 14 and 16 to gradually increase.

In this case, too, an overall imbalance between the containers 14 or 16 and the expected value, set in the weighing unit 15, causes the intervention of the processor 21, which controls the average pressure value inside the hermetic chamber 8a by increasing it or reducing it and accordingly controls the flow-rate of ultrafiltrate that the filter 4 is capable of producing.

It has thus been observed that the described invention achieves the intended aim and objects, i.e., that it allows a sort of automatic control of the pressure inside the filter 4 as a function of a value which can be preset by an operator and of the control of the fluid balance in a patient subjected to hemodialysis, hemofiltration and hemodiafiltration therapies, without resorting to additional pumps provided specifically to perform the control.

The invention thus conceived is susceptible of numerous modifications and variations, all of which are within the scope of the inventive concept.

All the details may furthermore be replaced with other technically equivalent elements.

In the practical embodiment of the invention, the materials employed, as well as the shapes and the dimensions, may be any according to the requirements without thereby abandoning the scope of protection of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method for controlling the fluid balance of a patient subjected to single-needle hemodialysis, comprising a first step for extracting blood to be purified from the patient, followed by a second step for the filtration and weight-based collection of the filtrate and by a third step for returning the purified blood to the patient, characterized in that during said second filtration step the average filtration pressure can be changed on command from a normal value to an ultrafiltration value and vice versa, said average value being in turn correlatable with a treatment completion time, a programmed weight change of the patient, said average pressure value being the result of corresponding limit values, a higher value and a lower value, which can be preset as required in an associated electronic logic control unit.

2. An apparatus for performing the method according to claim 1, comprising a first line for extracting blood from a patient through a single vascular access point, said line being controlled by a first peristaltic pump that operates intermittently and sends the blood cyclically into a filter, from which said blood exits in a purified state in order to be returned to the patient through a second infusion line, arranged in series to said first line and leading into said vascular access point, and which is controlled by a second peristaltic pump that operates intermittently in strict alternation with said first pump, at least one third line being also connected to said filter to discharge the filtrate into containers that can be coupled to a unit for weighing containers of infusion or dialysis fluids that are also coupled to said unit and are connected to said first extraction line; characterized in that a means for activating the alternations in the operation of said first and second pumps is interposed between said filter and said second pump, said means being adapted to control a pressure value that is the average of two presettable values, respectively a minimum value and a maximum value, inside said filter, modulating its flow-rate from a normal value to an ultrafiltration value and vice versa.

3. An apparatus according to claim 2, characterized in that said activation means is constituted by a hermetic chamber connected to said filter and to a corresponding pressure sensor, said chamber containing a volume of incompressible liquid and a volume of air that are in mutual dynamic equilibrium.

4. An apparatus according to claim 2, characterized in that access and insertion means and connection means for instruments for detecting the pressure in said first line are optionally provided on said first extraction line, upstream and/or downstream of the connection to the third line that originates from the containers of infusion or dialysis liquid.

5. An apparatus according to claim 2, characterized in that clamp means are mounted downstream of said second pump along the second line for return to the patient, said clamp means acting intermittently and being active in phase opposition with respect to said second pump, which is adapted to prevent reverse flows of blood towards the patient.

6. An apparatus according to claim 2, characterized in that a third peristaltic pump is mounted on said third line for connecting the containers of infusion liquid to the first extraction line, a conventional in-line air detector being installed upstream of said third pump, a heating element being mounted downstream of said third pump.

7. An apparatus according to claim 6, characterized in that a one-way valve is provided between said heating element and said first extraction line.
